# EUROPÄISCHE PATENTSCHRIFT

(11) **EP 2 409 645 B1**
(45) Veröffentlichungstag und Bekanntmachung des Hinweises auf die Patenterteilung: **14.11.2018**
(21) Anmeldenummer: 11173039.6
(22) Anmeldetag: 07.07.2011
(51) Int. Cl.: A61B 6/00, A61B 6/12, G06T 7/00, A61B 6/02

(54) **Bildunterstützte Biopsieentnahme**
Image-supported biopsy removal
Biopsie assistée par imagerie

(30) Priorität: 21.07.2010 DE 102010031738
(43) Veröffentlichungstag der Anmeldung: 25.01.2012
(73) Patentinhaber: Siemens Healthcare GmbH, 91052 Erlangen (DE)
(72) Erfinder: Fischer, Daniel, 91052 Erlangen (DE)

(56) Entgegenhaltungen:
- WO-A1-2006/039809
- WO-A1-2006/039809
- WO-A2-2007/095330
- DE-A1-102007 061 592
- DE-A1-102008 006 358
- DE-A1-102008 050 570
- US-A- 4 691 333
- US-A1- 2007 083 101
- US-A1- 2008 198 966
- MANUEL MEILINGER ET AL: "Alignment correction during metal artifact reduction for CBCT using mutual information and edge filtering", IMAGE AND SIGNAL PROCESSING AND ANALYSIS, 2009. ISPA 2009. PROCEEDINGS OF 6TH INTERNATIONAL SYMPOSIUM ON, IEEE, PISCATAWAY, NJ, USA, 16 September 2009 (2009-09-16), pages 135-140, XP031552078, ISBN: 978-953-184-135-1
- MEILINGER M ET AL: "Metal Artifact Reduction in CBCT Using Forward Projected Reconstruction Information and Mutual Information Realignment", WORLD CONGRESS ON MEDICAL PHYSICS AND BIOMEDICAL ENGINEERING,, vol. 25/2, 1 September 2009 (2009-09-01), pages 46-49, XP009154169,

## Beschreibung

Bei einer Biopsie wird eine Gewebeprobe, beispielsweise aus dem Gewebe der Mamma eines Patienten, zur histologischen Untersuchung entnommen. Eine Entnahme der Gewebeprobe kann unter Zuhilfenahme von bildgebenden Verfahren erfolgen. Mit Hilfe eines Tomosyntheseverfahrens wird ein Übersichtsvolumensatz von der Brust erstellt. Zur Erstellung des Übersichtsvolumensatzes werden Röntgenbilder von der Mamma aufgenommen. Zur Röntgenbildaufnahme wird eine Röntgenröhre in beispielsweise einer Kreisbahn über einen Detektor bewegt. Die Röntgenröhre bewegt sich beispielsweise bei der Tomosynthese in einem Winkelbereich von +25° bis -25°. In bestimmbaren Abständen wird dann eine Röntgenstrahlung in der Röntgenquelle ausgelöst und das jeweilige Röntgenbild aus dem Detektor ausgelesen und zwischengespeichert. Anschließend wird mit Hilfe eines Rekonstruktionsverfahrens ein Übersichtsvolumensatz erstellt. Anhand der Schichtbilder des Übersichtsvolumensatzes, werden Bereiche für die Entnahme einer Gewebeprobe lokalisiert und daraus die Koordinaten für die Einstichposition, -richtung und Einstichtiefe einer Biopsienadel ermittelt. Zur Gewebeentnahme wird die Biopsienadel in die Brust eingebracht. Da eine exakte Fixierung der Brust zwischen dem Detektor und einer Kompressionsplatte über die gesamte Untersuchungsdauer sehr schwer einzuhalten ist und die zu entnehmende Gewebeveränderung oft nur wenige Kubikmillimeter groß ist, wird vor der Entnahme der Gewebeprobe ein Kontrollvolumensatz von einer Mamma mit einer in das Brustgewebe eingeführten Biopsienadel erstellt. Mittels Auswertung der Schichtbilder des Kontrollvolumensatzes kann die Ausrichtung sowie die Position der Biopsienadelspitze überprüft und eine Anpassung und Ausrichtung der Biopsienadel vorgenommen werden. Die Überprüfung und Anpassung der Ausrichtung der Biopsienadelspitze anhand der Schichtbilder des Kontrollvolumensatzes erschwert sich durch sogenannte Out-of-Plane Artefakte.

Aus WO 2007/095330 A2 ist ein Mammographiegerät gemäß dem Oberbegriff des Patentanspruchs 1 bekannt.

US 2007/0083101 A1 beschreibt ein Verfahren zur Reduzierung von Artefakten bei der Erzeugung von dreidimensionalen Bilddaten, wobei aus unterschiedlichen Richtungen aufgenommene Rohdaten miteinander kombiniert werden.

Ein weiteres Verfahren zur Erzeugung von artefaktreduzierten 3-D-Bilddaten ist aus DE 10 2008 050 570 A1 bekannt. Hierbei wird ein Röntgenstrahlabbildung verfälschender 3-D- Bereich identifiziert, in dem die 3-D-Bilddaten durch Modelldaten eines für Röntgenstrahlen durchlässigen Mediums ersetzt werden. Hieraus werden 2-D-Bilder durch Vorwärtsprojektion ermittelt, die zur Korrektur der ursprünglichen 2-D-Bilder dienen. Zur Korrektur werden insbesondere Korrekturbereiche in den 2-D-Bildern ersetzt und anschließend en weiterer 3-D-Bilddatensatz erneut rekonstruiert.

US 2008/0198966 A1 versucht Artefakte bei einer bildüberwachten Biopsie durch eine geschickte Positionierung der Biopsienadel zu vermeiden. Gegebenenfalls kann der Bereich der Biospienadel von der Rekonstruktion des dreidimensionalen Volumensatzes ausgeschlossen werden oder ein zuvor aufgenommener Volumensatz ohne Biospienadel angezeigt werden.

Der Erfindung liegt die Aufgabe zugrunde, eine Vorrichtung für eine bildüberwachte Biopsieentnahme ohne den oben angegebenen Nachteil anzugeben.

Die Aufgabe wird durch die Merkmale des Patentanspruchs 1 gelöst.

Bei dieser Vorrichtung liegt ein Mammographiegerät mit Biopsieeinheit vor, bei dem ein erster Volumensatz ohne und ein zweiter Volumensatz mit Biopsienadel im Gewebe einer Mamma erstellt werden.

Eine Markierungseinheit markiert in Röntgenbildern zur Erstellung des zweiten Volumensatzes abgebildete Teile der Biopsieeinheit. Mittels einer Einfügeeinheit werden die markierten Bereiche in den Röntgenbildern für den zweiten Volumensatz durch Röntgenbilddaten von korrespondierenden Röntgenbildern für den ersten Volumensatz ersetzt.

Die Erfindung bringt den Vorteil mit sich, das Out-of-Plane-Artefakte vermieden werden.

Die Erfindung bringt den Vorteil mit sich, dass in den bereinigten Röntgenbilddaten für den zweiten Volumensatz die Biopsienadel nachgebildet wird.

Die Erfindung bringt den Vorteil mit sich, dass die Darstellung des Brustgewebes klar und deutlich ist.

Nachfolgend wird der Gegenstand der Erfindung anhand von Figuren eines Ausführungsbeispiels näher erläutert.

Es zeigen:
Figur 1 eine Frontansicht von einer Biopsieeinheit,
Figur 2 einen Projektionsverlauf einer Röntgeneinheit,
Figur 3 eine Draufsicht auf die Biopsieeinheit,
Figur 4 eine weitere Draufsicht und
Figur 5 ein Blockdiagramm.

Bei dieser Vorrichtung und dem dazugehörigen Verfahren werden Abbildungen von einer Biopsieeinheit in Röntgenbildern für einen zweiten Volumensatz ermittelt und durch Röntgenbilddaten von korrespondierenden Röntgenbildern für einen ersten Volumensatz ersetzt und ein überarbeiteter zweiter Volumensatz erstellt.

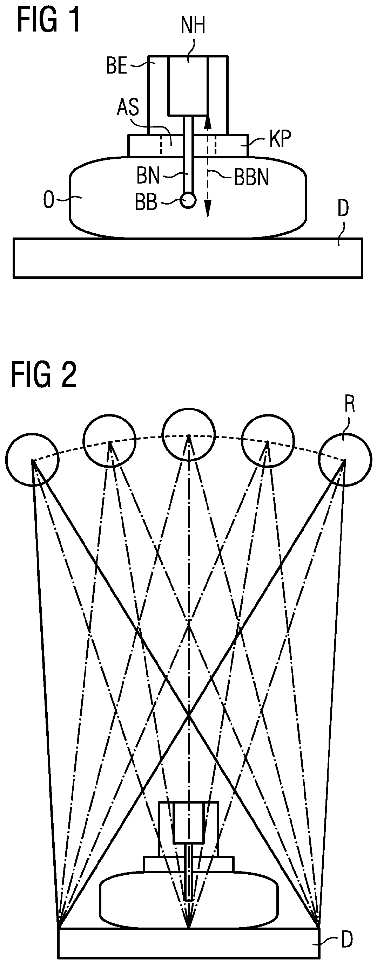

Figur 1 zeigt ein zwischen Kompressionsplatte KP und Oberfläche eines Detektors D komprimiertes und fixiertes Objekt O. Dieses Objekt O kann beispielsweise die Brust eines Patienten sein. Die Biopsienadel BN ist über einen Biopsienadelhalter NH mit der Positioniereinheit BE verbunden. Mit der Positioniereinheit BE wird die Biopsienadel BN auf eine lokalisierte Gewebeveränderung ausgerichtet. Diese Gewebeveränderung kann beispielsweise in Schichtbildern aus dem ersten Volumensatz, der auch als Übersichtsvolumensatz bezeichnet wird, mittels einem Bildauswertealgorithmus lokalisiert werden. Die Biopsienadel BN wird nach einer Priorisierung der lokalisierten Gewebeveränderungen rechnergesteuert auf eine ausgewählte Gewebeveränderung navigiert und ausgerichtet. Die Biopsienadel BN wird dazu innerhalb einer Aussparung AS der Kompressionsplatte KP auf die Gewebeveränderung ausgerichtet. In Figur 1 ist die Biopsienadel BN in das Gewebe der Brust eingeführt und auf eine Gewebeveränderung ausgerichtet. Die Spitze der Biopsienadel wird unmittelbar vor der lokalisierten Gewebeveränderung positioniert. Erst nach einer Überprüfung, ob die Biopsienadel richtig ausgerichtet ist, erfolgt die Entnahme der Gewebeprobe.

In Figur 2 ist eine Trajektorie einer Röntgenröhre R eines Mammographiegerätes wiedergegeben. Die Trajektorie kann dabei wie dargestellt entlang eines Kreissegmentes oder innerhalb einer Ebene erfolgen. Für eine Tomosynthese wird eine erste Sequenz von beispielsweise 25 Röntgenbildern erstellt. Dabei wird die Röntgenröhre R über dem Detektor D entlang eines Kreisbogens in einem Winkelbereich zwischen 25° bis -25° bewegt. Objekte unterschiedlicher Höhe in der Brust werden bei verschiedenen Winkeln unterschiedlich auf den Detektor projiziert. Während der anschließenden Rekonstruktion, zum Beispiel durch die Methode der gefilterten Rückprojektion, werden auffällige Gewebestrukturen in der Mamma durch geeignetes Filtern, Verschieben und Summieren verstärkt. Die Rekonstruktion führt zu einer Reihe von Schichtenbildern in unterschiedlichen Tiefenlagen parallel zur Detektoroberfläche. Bei dem beschriebenen Ausführungsbeispiel werden eine erste Sequenz ST1 von Röntgenbildern von der komprimierten Brust sowie eine zweite Sequenz ST2 von Röntgenbildern vor einer Biopsieentnahme aus dem Gewebe der Brust angefertigt. Mit der ersten Sequenz ST1 wird ein erster Volumensatz ÜVS, ein Übersichtsvolumensatz und mit der zweiten Sequenz ST2 wird ein zweiter Volumensatz KVS, ein Kontrollvolumensatz, erstellt. Von Nachteil ist, dass bei den Schichtbildern aus dem Tomosynthese-Rekonstruktionsprozess Artefakte durch die Abbildung von Biopsienadel, Biopsienadelhalter oder Biopsienadelhalter mit Positioniereinheit in den einzelnen Röntgenaufnahmen entstehen. Der erfinderische Gedanke liegt nun darin, Abbildungen von Biopsienadel BN, Biopsienadelhalter NH und Teile der Positioniereinheit BE in den einzelnen Röntgenbilddatensätzen bzw. Projektionen der zweiten Sequenz zu erkennen und zu extrahieren und durch Röntgenbilddaten aus Röntgenbildsätzen aus der ersten Sequenz zu ersetzen.

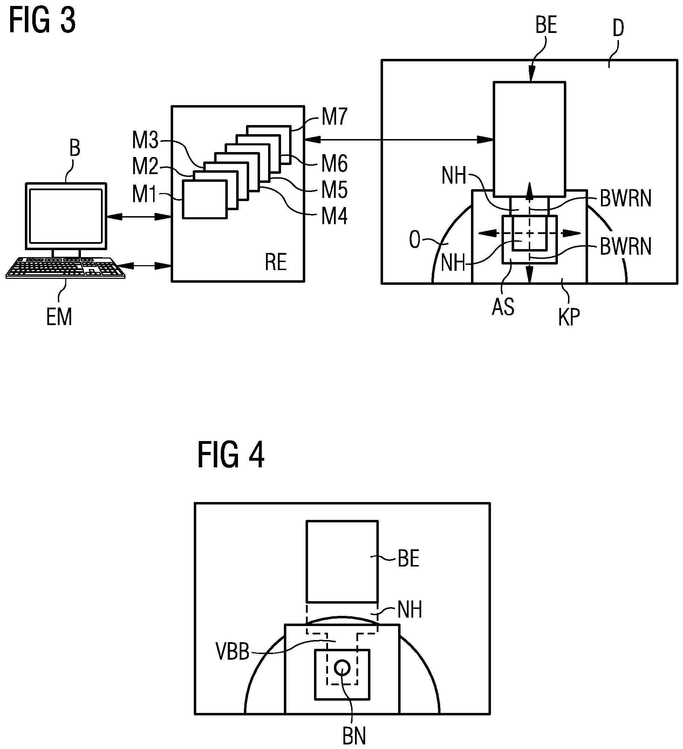

In Figur 3 ist eine Draufsicht auf die Biopsieeinheit BE, NH gezeigt. Sowohl die Erstellung des Übersichtsvolumensatzes ÜVS, des Kontrollvolumensatzes KVS, die Vorpositionierung der Biopsienadel BN, die exakte Ausrichtung der Biopsienadel sowie die Entnahme des Bioptates wird über die mit der Biopsieeinheit BE verbundenen Recheneinheit RE durchgeführt.

Bei dieser Ausgestaltung sind die einzelnen oben aufgeführten Verarbeitungseinheiten bzw. Verarbeitungsschritte in den Modulen M1 bis M7 der Recheneinheit RE integriert. So wird im ersten Modul M1 die Erstellung einer ersten Sequenz ST1 von Röntgenaufnahmen initiiert. In einem zweiten Modul M2 ist eine Einheit zur Erstellung eines Übersichtsvolumensatzes, ein erster Volumensatz ÜVS, sowie eine Ausleseinheit STST1 zum Auslesen von Schichtbildern integriert. In dem nachfolgenden dritten Modul M3 ist eine Lokalisiereinheit LE, eine Koordinatenermittlungseinheit PE für lokalisierte Gewebeveränderungen sowie eine Bereitstellungseinheit AB zur Abgabe von Steuerdaten für die Ausrichtung und Einführung der Biopsienadel BN angeordnet. Im vierten Modul M4 wird ein Ablauf einer zweiten Sequenz ST2 von Röntgenbildern angestoßen und ein Kontrollvolumensatz KVS errechnet. Im fünften Modul M5 ist eine Markiereinheit MA sowie eine Ermittlungseinheit KR zur Ermittlung von korrespondierenden Röntgenbildern in der ersten und zweiten Sequenz ST1, ST2 integriert. Im sechsten Modul M6 ist eine Einfügeeinheit EET zur Erstellung von bereinigten Röntgenbilddaten angeordnet. In diesem Modul werden Röntgenbilddaten aus der ersten Sequenz ST1 in korrespondierenden Röntgenbildern aus der zweiten Sequenz ST2 eingefügt. Ersetzt werden die Röntgenbildteile in den Röntgenbildern der zweiten Sequenz ST2 bei denen Biopsienadel BN, Biopsienadelhalter NH und Teile der Positioniereinheit BE abgebildet sind durch Röntgenbilddaten aus der ersten Sequenz ST1. Im siebten Modul M7 wird die Berechnung eines bereinigten Kontrollvolumensatzes BVS durchgeführt. In dem bereinigten Kontrollvolumensatz BVS wird dann die Biopsienadel BN entsprechend ihrer Position und Ausrichtung virtuell eingefügt.

Mit der Recheneinheit RE sind Eingabemittel EM sowie eine Bildschirmeinheit B verbunden. Die Biopsieeinheit, besteht unter anderem aus der Biopsienadel BN, dem Biopsienadelhalter NH sowie einer Positioniereinheit BE für den Biopsienadelhalter NH. Die Brust wird entsprechend der Lokalisation einer zu untersuchenden Gewebeveränderung derart unter der Kompressionsplatte positioniert, dass sich die Gewebeveränderung unterhalb der Aussparung AS befindet. Zur Feinabstimmung bzw. Ausrichtung der Biopsienadel wird diese mit Hilfe der von der Recheneinheit RE bereitgestellten Steuersignale positioniert. Die Soll-Positionierung und die aktuelle Positionierung der Biopsienadelspitze werden mittels der Recheneinheit RE verglichen. Die Ansteuereinheit BE steuert die Biopsienadel BN entsprechend den angezeigten Bewegungsrichtungen BWRN, BBN bis die Biopsienadelspitze exakt auf die Gewebeveränderung ausgerichtet ist. Zur Gewebeentnahme wird die Spitze der Biopsienadel bis wenige Millimeter vor der lokalisierten Gewebeveränderung in das Gewebe der Brust eingeführt. Zur Kontrolle wird vor der Entnahme des Gewebes eine zweite Sequenz ST2 von Röntgenaufnahmen für den Tomosynthese-Rekonstruktionsprozess aufgenommen.

In Figur 4 ist eine Draufsicht auf die Positioniereinheit BE für den Biopsienadelhalter NH und die Biopsienadel BW gezeigt. Die gestrichelte Linie gibt mögliche Ausmaße des Biopsienadelhalters NH wieder. Während der Röntgenaufnahmen werden Teile des Biopsienadelhalters NH sowie die Biopsienadel BN auf diesen abgebildet.

Die Brust wird in der Kompressionseinheit des Mammographiegerätes fixiert und komprimiert. In dieser verbleibt die Brust bis zur Biopsieentnahme.

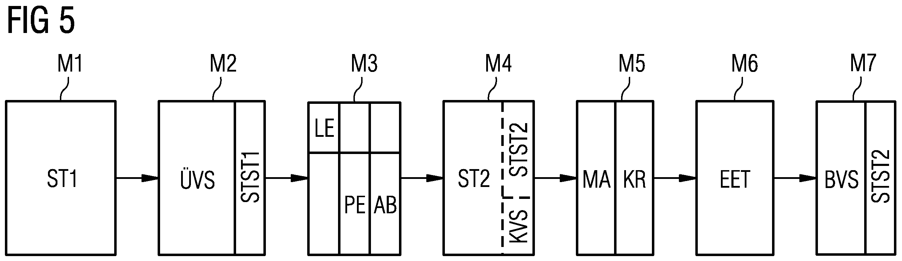

In Fig. 5 ist ein Blockschaltbild zur Vermeidung von Out-of-Plane-Artefakten bei einem Tomosynthese-Rekonstruktionsprozess abgebildet. Die jeweils zur Begutachtung anstehenden Schichtbilder werden aus einem zweiten überarbeiten Volumensatz BVS abgerufen. In den einzelnen Modulen M1 bis M7 sind schematisch Einheiten bzw. Verarbeitungsprozeduren zusammengefasst. Im ersten Modul M1 wird die Erstellung einer ersten Sequenz von Röntgenaufnahmen bzw. Projektionen ST1 für einen ersten Tomosynthese-Rekonstruktionsprozess initiiert. Die Daten daraus werden in dem zweiten Modul M2 in einem ersten Volumensatz ÜVS, der auch als Übersichtsvolumensatz bezeichnet wird, zusammengefasst. Aus diesem können dann eine Vielzahl von Schichtbildern STST1 abgerufen werden. Im dritten Modul M3 sind eine Lokalisiereinheit LE, eine Positioniereinheit PE sowie eine Ausrichteinheit AB integriert. Mit diesem dritten Modul M3 werden die Lokalisationen von Gewebeveränderungen in der Mamma bestimmt. Die Biopsienadel wird in die Brust bis unmittelbar vor die Gewebeveränderung eingeführt. Im vierten Modul M4 wird eine zweite Sequenz von Röntgenaufnahmen ST2 für einen zweiten Tomosynthese-Rekonstruktionsprozess initiiert und durchgeführt. Mit diesen Schichtbildern wird die Position und Ausrichtung der in die Brust eingeführten Biopsienadel BN kontrolliert. Im fünften Modul M5 werden die Bereiche in den Projektionsbildern, die durch Biopsienadel, Nadelhalter oder Positioniereinheit BE verdeckt worden sind, markiert. Die Markierung kann mit Hilfe eines Segmentierungsalgorithmus erfolgen. Die Markierung der Bereiche in den jeweiligen Röntgenbildern kann basierend auf die Position und Form der Biopsienadel des Nadelhalters und der geometrischen Gegebenheiten von Röntgenkopf und Detektoreinheit erfolgen. Durch das Einführen der Biopsienadel kann das Gewebe in der Brust deformiert werden. Um den Rechenaufwand von einem Registrier- oder Mappingverfahren zwischen Röntgenbildern aus der ersten und zweiten Sequenz ST1, ST2 gering zu halten, werden die Röntgenbilddaten im Umfeld der eingeführten Biopsienadel entsprechend durch einen Deformationsalgorithmus angepasst. In einer Korrespondiereinheit KR werden in einem weiteren Schritt jeweils die korrespondierenden Röntgenbilder aus der ersten und zweiten Sequenz ST1, ST2 ermittelt. Dies kann durch registrierende Verfahren oder einem Bildmappingverfahren erfolgen. Sind die Röntgenbildpaare ermittelt, so werden mittels der im sechsten Modul M6 angeordneten Einfügeeinheit EET Röntgenbilddaten aus den markierten Bereichen der zweiten Sequenz ST2 durch entsprechende Bereiche aus Röntgenbilddaten der ersten Sequenz ST1 ersetzt. Der markierte Bereich kann auch um einen Sicherheitsabstand vergrößert werden. Die Daten aus den Röntgenbildern, die ohne Biopsienadel, Biopsiehalter und/oder Biopsieeinheit erstellt wurden, können gefiltert werden, bevor sie eingefügt bzw. nachdem sie eingefügt wurden. Die Röntgenbilder, die mit und ohne Biopsienadel erstellt wurden, können miteinander registriert werden. Zur Registrierung der Röntgenbilder können auch Projektionen, die unter einem anderen Winkel aufgenommen wurden, verwendet werden. Während der Registrierung kann mittels eines Deformationsmodells die Verschiebung des Gewebes bei eingeführter Biopsienadel zu berücksichtigen. Die unmarkierten Bereiche können um das Rauschen zu reduzieren mit den Projektionsbildern ohne Biopsienadel gemischt werden. Im siebten Modul M7 wird ein überarbeiteter zweiter Volumensatz BVS ermittelt. Die Rekonstruktion mit den überarbeiteten Röntgenbilddaten wird dann angestoßen, nachdem die markierten Bereiche in den Röntgenbildern der zweiten Sequenz ST2 ersetzt und evtl. auch eine Gewebeverschiebung durch ein Deformationsmodell berücksichtigt wurden.

Abbildungen von der Biopsienadel BN, dem Nadelhalter NH und/oder Teilen der Positioniereinheit BE können nach der Rekonstruktion in die Schichtbilder eingefügt werden. Zur Abbildung der einzelnen Gegenstände kann auf 3D-Modeldaten zurückgegriffen werden. Die Konturen der Biopsienadel BN können aber auch separat rekonstruiert werden. Die in den Voraufnahmen festgelegte Position des zu biopsierenden Bereichs wird in den Schichtbildern angezeigt.

Anstelle von Tomosynthese-Projektionen können auch ein erstes stereotaktische Bildpaare zur Lokalisation der Gewebeveränderung und ein zweites stereotaktisches Bildpaar zur Positionierungskontrolle der Biopsienadel erstellt werden. Auch hier können die in den Röntgenaufnahmen für das zweite stereotaktische Bildpaar abgebildeten Teile der Biopsienadel durch entsprechende Teile der Röntgenaufnahmen für das erste stereotaktische Bildpaar ersetzt werden.

### Bezugszeichenliste:

- R: Röntgenquelle
- D: Detektor
- O: Objekt
- BB: Gewebeveränderung
- BE: Positioniereinheit
- BN: Biopsienadel
- NH: Biopsienadelhalter
- BBN: Bewegungsrichtung der Biopsienadel
- RE: Recheneinheit
- B: Bildschirmeinheit
- EM: Eingabeeinheit
- M1, ..., M7: erstes bis siebtes Modul
- KP: Kompressionsplatte
- AS: Aussparung
- ST1: erste Sequenz von Röntgenaufnahmen
- ÜVS: erster Volumensatz/ Übersichtsvolumenbild
- STST1: Schichtbilder aus erstem Volumensatz
- RK1: erste Rekonstruktion
- ST2: zweiten Sequenz von Röntgenaufnahmen
- KVS: zweiter Volumensatz/ Kontrollvolumenbild
- STST2: Schichtbilder aus zweitem Volumensatz
- LE: Lokalisiereinheit
- PE: Koordinatenermittlungseinheit
- AB: Bereitstellungseinheit
- MA: Markierungseinheit
- KR: Ermittlungseinheit von korrespondierenden Röntgenbildern
- EET: Einfügeeinheit
- BVS: bereinigter zweiter Volumensatz

## Patentansprüche

1. Mammographiegerät mit Biopsieeinheit (BE, NH, BN), welches zur Erzeugung eines ersten Volumensatzes (ÜVS) sowie zur Erzeugung eines zweiten Volumensatzes (KVS, BVS), jeweils aus Röntgenbilddaten aufweisende Projektionsbildern, ausgebildet ist, wobei der erste Volumensatz (ÜVS) ohne und der zweite Volumensatz (KVS) mit einem Einsatz der Biopsieeinheit(BE, NH, BN) erstellt wird und eine Markierungseinheit (MA) zur Markierung von durch eine Biopsienadel (BH), einen Nadelhalter (NH) oder eine Positioniereinheit (BE) verdeckten Bereichen in den Projektionsbildern für den zweiten Volumensatz (KVS, BVS) umfasst, **gekennzeichnet durch** eine Einfügeeinheit (EET) zur Einfügung der Röntgenbilddaten in die markierten Bereiche, wobei die markierten Bereiche in den Projektionsbildern für den zweiten Volumensatz (KVS, BVS) durch die Röntgenbilddaten von korrespondierenden Projektionsbildern für den ersten Volumensatz (ÜVS) ersetzt werden.

2. Mammographiegerät nach Patentanspruch 1,
**dadurch gekennzeichnet,**
**dass** dieses eine Ermittlungseinheit (KR) zur Ermittlung von jeweils korrespondierenden Projektionsbildern für den ersten und zweiten Volumensatz aufweist.

3. Mammographiegerät nach einem der vorhergehenden Patentansprüche,
**dadurch gekennzeichnet,**
**dass** dieses eine Koordinatenermittlungseinheit (PE) zur Ausrichtung der Biopsienadel (BN) der Biopsieeinheit (BE, NH, BN) auf eine lokalisierte Gewebeveränderung aufweist.

4. Mammographiegerät nach einem der vorhergehenden Patentansprüche,
**dadurch gekennzeichnet,**
**dass** dieses eine Einheit (M7) zur Erstellung eines überarbeiteten zweiten Volumensatzes (BVS) aufweist, in dem die aktuelle Position und Form der Biopsienadel (BN) durch 3D-Modelldaten eingefügt sind.

## Claims

1. Mammography device with biopsy unit (BE, NH, BN) which is designed to generate a first volume set (ÜVS) and to generate a second volume set (KVS, BVS), in each case from projection images comprising X-ray image data, wherein the first volume set (ÜVS) is created without and the second volume set (KVS) is created with use of the biopsy unit (BE, NH, BN), and comprises a marking unit (MA) for marking regions in the projection images hidden by a biopsy needle (BH), a needle holder (NH) or a positioning unit (BE) for the second volume set (KVS, BVS), **characterised by** an insertion unit (EET) for insertion of the X-ray image data into the marked regions, wherein the marked regions in the projection images for the second volume set (KVS, BVS) are replaced by the X-ray image data from corresponding projection images for the first volume set (ÜVS).

2. Mammography device according to claim 1,
**characterised in that**
it has a determination unit (KR) for determining respective corresponding projection images for the first and second volume set.

3. Mammography device according to one of the preceding claims, **characterised in that**
it has a coordinate determination unit (PE) for aligning the biopsy needle (BN) of the biopsy unit (BE, NH, BN) to a localised tissue alteration.

4. Mammography device according to one of the preceding claims, **characterised in that**
it has a unit (M7) for creating a revised second volume set (BVS), in which the current position and shape of the biopsy needle (BN) are inserted using 3D model data.

## Revendications

1. Appareil de mammographie ayant une unité (BE, NH, BN) de biopsie, constitué pour produire un premier jeu (ÜVS) de volume ainsi que pour produire un deuxième jeu (KVS, BVS) de volume, constitués chacun d'images de projection ayant des données de radiographie, le premier jeu (ÜVS) de volume étant établi sans et le deuxième jeu (KVS) de volume avec une utilisation de l'unité (BE, NH, BN) de biopsie, et comprenant une unité (MA) de repérage pour repérer des parties, recouvertes par une aiguille (BH) de biopsie, un porte-aiguille (NH) ou une unité (BE) de mise en position, dans les images de projection pour le deuxième jeu (KVS, BVS) de volume, **caractérisé par** une unité (EET) d'insertion pour l'insertion des données de radiographie dans les parties repérées, les parties repérées dans les images de projection étant, sur le deuxième jeu (KVS, BVS) de volume, remplacées par les données de radiographie d'images de projection correspondantes sur le premier jeu (ÜVS) de volume.

2. Appareil de mammographie suivant la revendication 1, **caractérisé**
**en ce que** celui-ci a un unité (KR) de détermination pour déterminer des images de projection correspondantes respectivement sur le premier et le deuxième jeu de volume.

3. Appareil de mammographie suivant l'une des revendications précédentes,
**caractérisé**
**en ce que** celui-ci a une unité (PE) de détermination de coordonnées pour l'orientation de l'aiguille (BN) de biopsie de l'unité (BE, NH, BN) de biopsie sur une modification de tissu localisée.

4. Appareil de mammographie suivant l'une des revendications précédentes,
**caractérisé**
**en ce que** celui-ci a une unité (M7) d'établissement d'un deuxième jeu (BVS) de volume surtravaillé, dans lequel la position en cours et la forme de l'aiguille (BN) de biopsie est insérée par des données de modèle en 3D.
